(19) Europäisches Patentamt / European Patent Office / Office européen des brevets

(11) **EP 3 609 583 B1**

(12) **EUROPÄISCHE PATENTSCHRIFT**

(45) Veröffentlichungstag und Bekanntmachung des Hinweises auf die Patenterteilung:
**12.02.2025 Patentblatt 2025/07**

(21) Anmeldenummer: **18725746.4**

(22) Anmeldetag: **10.04.2018**

(51) Internationale Patentklassifikation (IPC):
*A61Q 11/00* (2006.01)   *A61K 8/02* (2006.01)
*A61K 8/73* (2006.01)

(52) Gemeinsame Patentklassifikation (CPC):
**A61Q 11/00; A61K 8/025; A61K 8/027;**
**A61K 8/731;** A61K 2800/28; A61K 2800/412

(86) Internationale Anmeldenummer:
**PCT/EP2018/059111**

(87) Internationale Veröffentlichungsnummer:
**WO 2018/189149 (18.10.2018 Gazette 2018/42)**

(54) **ZAHNPFLEGEMITTEL MIT NATIVER CELLULOSE**

DENTAL CARE WITH NATIVE CELLULOSE

SOINS DENTAIRES AVEC CELLULOSE NATIVE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **10.04.2017 DE 102017107654**

(43) Veröffentlichungstag der Anmeldung:
**19.02.2020 Patentblatt 2020/08**

(73) Patentinhaber: **Dentacare Gmbh Institut Für Moderne Zahnmedizin**
**64319 Pfungstadt (DE)**

(72) Erfinder:
• **JUNG, Andreas**
**64367 Mühltal (DE)**
• **JUNG, Thomas**
**64285 Darmstadt (DE)**
• **HAYAG, Hans Dieter**
**77652 Offenburg (DE)**

(74) Vertreter: **2K Patentanwälte Blasberg Kewitz & Reichel**
**Partnerschaft mbB**
**Schumannstrasse 27**
**60325 Frankfurt am Main (DE)**

(56) Entgegenhaltungen:
**EP-A1- 2 893 957    DE-A1- 4 113 044**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung ist in den beiliegenden Ansprüchen definiert und betrifft ein Mund- und Zahnpflege- sowie Zahnreinigungsmittel in Form einer Paste mit einem Zusatz von feinkörnigen, wasserunlöslichen Reinigungskörpern.

Die im Markt befindlichen herkömmlichen Mund- und Zahnpflege- sowie Zahnreinigungsmittel in Form einer Paste, auch kurz Zahnpasten genannt, beinhalten üblicherweise Inhaltsstoffe, welche einen Putz- und Abriebeffekt auf den Zähnen zu Folge hat. Quantitativ werden diese Effekte durch einen PCR-Wert (Reinigung; PCR = (Pellicle Cleaning Ratio) und einen RDA-Wert (Abrieb; RDA = Relative Dentin Abrasion) definiert. Somit werden bei jeder Anwendung die Zähne nicht nur gereinigt, sondern es wird auch ein leichter Abrieb des Zahnschmelzes verursacht. Die Zahnpasten enthalten üblicherweise Reinigungskörper (Schleifmittel) in Form von Silikat- oder Kieselgelteilchen, die aufgrund ihrer Härte und ihrer kantigen Oberflächenstruktur jedoch einen hohen Abriebwert aufweisen und den Zahnschmelz, vor allem bei sehr häufiger Zahnpflege, verhältnismäßig stark angreifen.

Aus der DE 10 2014 201 628 A1 wird ein Mund- und Zahnpflege- sowie Zahnreinigungsmittel in Form einer Paste offenbart, deren Reinigungskörper aus einem Zellstoff bestehen, der chemisch aufbereitet wurde, vorzugsweise aus mikrokristalliner Cellulose bestehen. Die Reinigungskörper haben eine kugelige, runde Geometrie sowie glatte Oberfläche mit einer Knollenstruktur. Damit soll eine gute und gleichzeitig schonende Zahnreinigung auch in schwer zugänglichen Zahnbereichen ermöglicht werden.

Aufgabe der vorliegenden Erfindung ist es, eine deutlich verbesserte Zahnpasta der eingangs genannten Art bereit zu stellen.

Gelöst wird die Aufgabe durch ein Mund- und Zahnpflege- sowie Zahnreinigungsmittel in Form einer Paste mit einem Zusatz von feinkörnigen, wasserunlöslichen Reinigungskörpern wie im Anspruch 1 definiert.

Demnach ist die Erfindung dadurch gekennzeichnet, dass die Reinigungskörper aus reiner nativer Cellulose bestehen, die einer unbehandelten, natürlichen Cellulose entspricht, die mittels Aufschlussverfahren aus pflanzlichem Vorläufermaterial gewonnenen wird und die im Gegensatz zu mikrokristalliner Cellulose keinem weiterem nachfolgendem chemisch-modifizierenden Prozess unterzogen wird, und als kurzfaserige Partikel mit einer unregelmäßigen Oberfläche und einer oberflächen-rauhen Struktur, die eine Vielzahl von Erhebungen und Vertiefungen aufweist, ausgebildet sind, und dass die Reinigungskörper eine durchschnittliche Partikelgröße von 30 μm bis 80 μm aufweisen.

Unter reiner nativer Cellulose wird unbehandelte, natürliche Cellulose verstanden, die aus pflanzlichem Vorläufermaterial mittels Aufschlussverfahren gewonnen wird und die im Gegensatz zu mikrokristalliner Cellulose (MCC) keinem weiteren nachfolgenden chemisch-modifizierenden Prozess unterzogen wird. Insbesondere wurde die rein native Cellulose nicht, wie bei der Herstellung von MCC üblich, mit verdünnter Saure unter Temperatureinwirkung (> 100°C) chemisch behandelt und/oder einer Carboxylierung unterzogen. Die erfindungsgemäß verwendete native Cellulose ist rein pflanzlichen Ursprungs und nicht chemisch aufbereitet worden. Daher wird die rein native Cellulose hier nachfolgend auch als natürliche Cellulose bezeichnet. Dabei weisen die aus der nativen Cellulose gebildeten Partikel / Reinigungskörper eine sphärische, kurzfaserige Form mit einer unregelmäßigen Oberfläche auf. Die durchschnittliche Partikelgröße beträgt 30-80 μm, vorzugweise etwa 50 μm.

Die Erfinder haben erkannt und durch intensive Laborversuche belegt, dass im Vergleich mit herkömmlichen Zahnpasten, welche mikrokristalline Cellulose enthalten, das hier offenbarte Mund- und Zahnpflege- sowie Zahnreinigungsmittel durch Einsatz von rein nativer bzw. natürlicher Cellulose eine deutlich bessere Reinigungsleistung erzielt und dabei trotzdem einen geringeren Abrieb des Zahnschmelzes aufweist. Es werden also zwei an sich gegensätzlich zueinanderstehende Effekte bzw. Ziele erreicht, nämlich eine hohe Reinigungsleistung und ein geringer Abriebwert / RDA-Wert. Die Ergebnisse der Laborversuche werden in der nachfolgenden Beschreibung im Detail beschrieben.

Bevorzugte Ausgestaltungen ergeben sich aus den Unteransprüchen:

Demnach sind die Partikel der Reinigungskörper vorzugsweise sphärische Partikel, die bezüglich ihres Durchmessers eine Partikelgröße von etwa 50 μm aufweisen.

Die Reinigungskörper bestehen vorzugsweis aus reinen, weißen und geruchslosen Cellulose-Partikeln. Und der Anteil der Reinigungskörper in der Paste beträgt 10 bis 20 Gew.-%, vorzugsweise 14 Gew.-%.

Außerdem wird die Verwendung eines erfindungsgemäßen Mund- und Zahnpflege- sowie Zahnreinigungsmittel für die Reinigung von Zähnen, einschließlich von mit Multibandapparaturen versehenen Zähnen, offenbart. Ebenso die Verwendung von Körpern aus reiner nativer Cellulose als Reinigungskörper zur Reinigung von Zähnen, insbesondere zur Reinigung von mit Multibandapparaturen versehenen Zähnen.

Die Erfindung beruht auf der Erkenntnis, dass auch der Zusatz von feinkörnigen, wasserunlöslichen Reinigungskörpern aus natürlicher Cellulose in einer Paste für die Zahnreinigung und die Mund- und Zahnpflege die Zähne von schädlichem Zahnbelag insbesondere in engen Zahnzwischenräumen und in Bereichen unter dem Zahnfleischsaum beim Zähneputzen befreien kann, ohne dass dabei der Zahnschmelz angegriffen wird, was z. B. Messungen des RDA-Werts (Radioactive Dentine Abrasion) zeigen.

Der Erfindung liegt insbesondere der Gedanke zugrunde, dass sich die Härte der aus natürlichen Pflanzenfasern gewonnenen nativen Cellulose über einen weiten Bereich variieren und so auf eine für Reinigungskörper in einem Mund- und Zahnpflege- sowie Zahnreinigungsmittel günstige Härte einstellen lässt, bei der der Zahnschmelz während der Anwendung eines solchen Mittels geschont wird. Ein Vorteil der aus reiner nativer Cellulose bestehenden Reinigungskörper ist auch, dass diese keinerlei Toxizität oder Reizwirkung besitzen.

In Versuchen hat der Erfinder nämlich herausgefunden, dass Reinigungskörper aus reiner nativer Cellulose bei Wasser- und Ölzusatz nicht aufquellen.

Anders als Reinigungskörpern aus synthetischem Ethylen-Vinylacetat-Copolymerisat entziehen also die Reinigungskörper aus reiner nativer Cellulose in einem erfindungsgemäßen Mund- und Zahnpflege- sowie Zahnreinigungsmittel der Paste keine Wirkstoffe. Vielmehr gewährleisten die Reinigungskörper bzw. Partikel aus reiner nativer Cellulose, dass die in dem Mund- und Zahnpflege- sowie Zahnreinigungsmittel enthaltenen Wirkstoffe beim Zähneputzen zu den Zähnen, in das Zahnfleisch und in die Mundschleimhaut gelangen können.

Im Folgenden wird die Erfindung näher erläutert, wobei die folgende Beschreibung sich auf die Anwendung einer Zahnpaste (Zahncreme) bezieht, die folgende Formulierung aufweist, wobei die Summe aller Gewichtsanteile nicht mehr als 100% betragen dürfen:

| | |
|---|---|
| 50 bis 70 Gew.-% | Feuchthaltemittel, vorzugsweise etwa 55 Gew.-%; |
| 10 bis 20 Gew.-% | Reinigungskörper aus reiner nativer Cellulose vorzugsweise 14 Gew.-% in einer Partikelgröße von 30-80 $\mu$m, bevorzugt etwa 50 $\mu$m; |
| 25 bis 40 Gew.-% | Süßungsmittel, vorzugsweise Natriumsacharin und/oder Sorbitol |
| 1 bis 3 Gew.-% | Binde- und Verdickungsmittel, vorzugsweise Cellulose Gum und/oder Xanthan Gum |
| 2 bis 5 Gew.-% | Schaumbildner, vorzugsweise Tenside |
| 2 bis 5 Gew.-% | Wirkstoffe |
| 1 bis 3 Gew.-% | Aromatisierungsmittel |
| 0,1 bis 0,5 Gew.-% | Fluoride |

Die hier offenbarte Zahnpaste ist ein Mund- und Zahnpflege- sowie Zahnreinigungsmittel, bei dem die darin enthaltenen Reinigungskörper einen mittleren Teilchendurchmesser von etwa 50 $\mu$m aufweisen. Sie haben eine im Wesentlichen kugelige Gestalt mit einer glatten Oberfläche, die eine unregelmäßige Knollenstruktur hat.

Die Zahnpaste eignet sich u.a. auch zur Reinigung von mit Multibandapparaturen versehenen Zähnen. Derartige Apparaturen, die zur Korrektur von Zahnfehlstellungen dienen, weisen auf die Zähne aufgeklebte mechanische Aufnehmer (Brackets) auf, an denen Drahtbögen (Loops) angebracht und arretiert werden können.

Die mit den eine im Wesentlichen kugelige Gestalt aufweisenden Reinigungskörpern versehene Zahnpaste wird mittels einer üblichen Putztechnik, beispielsweise mit der Rotationsmethode angewendet. Durch die Einwirkung der Speichelflüssigkeit und das thixotrope Verhalten der Zahnpaste werden die in der Zahnpaste enthaltenen wasserunlöslichen Reinigungskörper beweglich.

Die von den Borsten der Zahnbürste angetriebenen Reinigungskörper vermitteln Schub- und Rotationsbewegungen an benachbarte Reinigungskörper. Dabei gelangen die Reinigungskörper auch an die nicht direkt von den Borsten erreichbaren Bereiche.

Die Reinigungswirkung der Reinigungskörper in der Zahnpaste besteht zum einen darin, dass die Rotationsbewegung zu einer Politur der Zahnoberfläche führt. Zum anderen dringen die Reinigungskörper direkt in die gröberen Verschmutzungen bzw. Ablagerungen ein und vergrößern deren Oberfläche. Dies führt zu einem Aufbrechen der Ablagerungen und zu einer erhöhten Wasserlöslichkeit, wodurch sich die Ablagerungen leichter entfernen lassen.

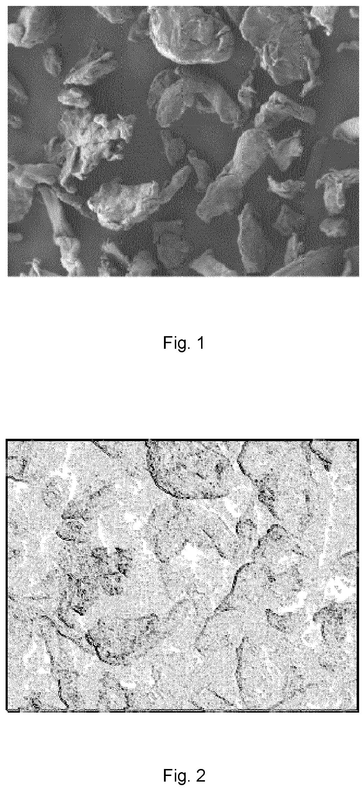

In den beiliegenden Figuren 1 und 2 wird die Struktur der aus nativer Cellulose bestehende Reinigungskörper bzw. -Partikel veranschaulicht. Die Fig. 1 zeigt eine mikroskopische Aufnahme / Fotografie; die Fig. 2 ist eine durch Kantendetektion gewonnene alternative Darstellung davon, welche die Form der Partikel noch deutlicher zeigt.

Die nicht als Knollenstruktur ausgebildete Oberfläche, sondern sphärische und kurzfaserige und oberflächen-rauhe Struktur der Reinigungskörper mit der darin ausgebildeten Vielzahl von Erhebungen und Vertiefungen bewirkt, dass beim Zähneputzen mit der Zahnpaste die Reinigungskörper auch in engen Fissuren mit der Oberfläche der Zähne in Kontakt geraten, so dass beim Zähneputzen dort ebenfalls eine gute Reinigungswirkung erzielt wird.

Durch die Anwendung der Reinigungskörper wird eine erhebliche Reibungsverminderung erreicht, so dass eine zusätzliche Kraft für das Bewegen der Reinigungskörper an den Zähnen zur Verfügung steht. Das Abrollen der Reinigungskörper hält dabei den Abrieb des Dentins beim Zähneputzen gering. Trotz der geringen Abrasivität des Mund-, Zahnpflege- und Zahnreinigungsmittels werden die Zähne damit bestens gereinigt und sind dann zungenglatt.

Im Rahmen von Untersuchungen hat der Erfinder festgestellt, dass die plaquereduzierende Reinigungswirkung der vorstehend beschriebenen Zahnpasta eine besonders effektive und doch sehr schonende (geringer RDA-Wert) Reinigungswirkung ermöglicht.

Durch intensive Laboruntersuchungen konnte belegt werden, dass die erfindungsgemäße Zahnpasta eine Reinigungswirkung von 84% und mehr erzielen kann, wo hingegen herkömmliche Zahnpasten mit MCC und Silica nur eine Reinigungswirkung von etwa 40% oder weniger erzielen (siehe nachfolgende Dokumentation).

Hinsichtlich des Abriebwertes kann die erfindungsgemäße Zahnpasta einen RDA-Wert von 8 und weniger erreichen, wo hingegen herkömmliche Zahnpasten mit MCC und Silica einen deutlich höheren RDA-Wert von etwa 32 aufweisen (siehe nachfolgende Dokumentation).

Das hier offenbarte Mund- und Zahnpflege- sowie Zahnreinigungsmittel in Form einer Paste ist biologisch abbaubar und erfüllt zudem vegane und andere Lebensmittel-Standards, wie z.B. auch die Voraussetzung das jüdische Koscher-Zertifikat und für das islamische Halal-Zertifikat.

Hierzu die Dokumentation der Laboruntersuchungen:

A) Zur Reinigungsleistung:

**Reinigungsleistung**

**Versuchsaufbau:**

- Herstellung einer Zahnpastaformel mit insgesamt 14% verschiedener Reinigungspartikel (auf Wasserbasis)
- Bestimmung der Reinigungsleistung am erhaltenen dritten molaren menschlichen Schmelz (in vitro) auf Basis von Stookey und Muhler (1968)[1] und Stookey et al. (1982)[2]
- Schmelzabschnitte wurden mit verminderter Körnung (1200 Körnung - 6 $\mu$m) für eine gleichmäßige Oberflächenlagerung gemahlen
- gelagert in Schwarztee bei 37 °C für 4 Stunden auf Basis von Addy und Moran (1995)[3]
- Suspension von 25 g Zahnpasta in 40 ml Wasser
- Einsatz des Bürstensimulators ZM-3.8 (SD-Mechatronic, Deutschland), Zahnbürste Oral-B Indicator 35 medium soft (Druck 150 g, 1.000 Hübe bei 60 mm sec$^{-1}$, Nabe 1 cm), 4 Messungen pro Probe

[1] Stookey. G. and Muhler, J. (1968): Laboratory Studies Concerning the Enamel and Dentin Abrasion Properties of Common Dentifrice Polishing Agents. J. Dent. Res. 47(4): 524-532.

[2] Stookey, G., Burkhard, T., Schemehorn, B. (1982): In Vitro Removal of Stain with Dentifries. J. Dent. Res. 61(11): 1236-1239.

[3] Addy, M. and Moran, J. (1995): Mechanisms of stain formation on teeth, in particular associated with metal ions and antiseptics, Adv. Dent. Res. 9(4): 450-456.

**Kolorimetrische Messungen:**

- fotografische Analyse der Proben vor und nach dem Färbeprozess sowie nach dem Reinigungsprozess
- farbmetrische Messungen zur Bestimmung von L*a*b-Werten (Spektrophometer CM 3600A, Konica Minolta) vor und nach dem Färbeprozess sowie nach dem Reinigungsprozess.

$\Delta E_1$ : kolorimetrischer Abstand zwischen der mit Strahlpulver behandelten Probenoberfläche und der gefärbten Probenoberfläche

$$\Delta E_1 = \sqrt{\Delta L_1^2 + \Delta a_1^2 + \Delta b_1^2}$$

$\Delta L_1$ = L nach Reinigungsvorgang - L nach Färbevorgang

$\Delta a_1$ = a nach Reinigungsvorgang - a nach Färbevorgang

$\Delta b_1$ = b nach Reinigungsvorgang - b nach Färbevorgang

$\Delta E_2$ : kolorimetrischer Abstand zwischen der ursprünglichen Probenoberfläche und der gefärbten Probenoberfläche

$$\Delta E_2 = \sqrt{\Delta L_2^2 + \Delta a_2^2 + \Delta b_2^2}$$

$\Delta L_2$ = ursprünglich - L nach Färbevorgang

$\Delta a_2$ = ursprünglich - a nach Färbevorgang

$\Delta b_2$ = ursprünglich - b nach Färbevorgang

$$[\%] = \frac{\Delta E_1}{\Delta E_2} * 100$$

**Reinigungsleistung**

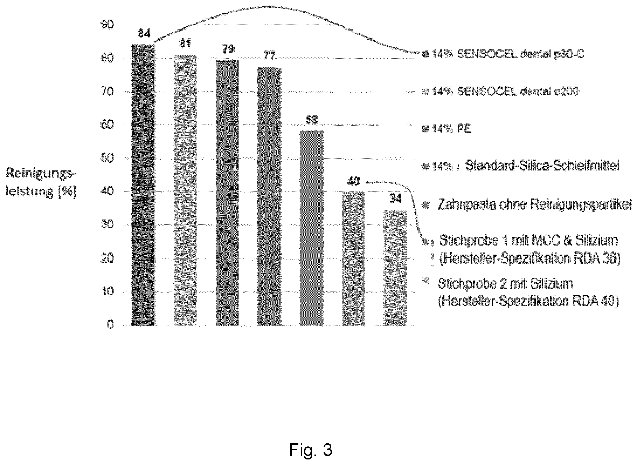

Eine Bewertung der Reinigungsleistung wird in der Fig. 3 veranschaulicht.

**Fazit:**

- Alle Testformeln sowie zwei Stichproben bekannter Zahnpasta-Marken wurden nach dem oben beschriebenen Versuchsaufbau getestet.

- SENSOCEL ® Dentalpartikel entsprechen der Reinigungswirkung von Standard-Silica (Kieselsäure) oder PE-Partikeln.

- SENSOCEL® Dentalartikel bieten eine sehr hohe Reinigungswirkung bei sehr geringem Abrieb.

- Durch den Einsatz von SENSOCEL Dentalpartikeln kann das optimale Verhältnis zwischen RDA und Reinigungsleistung erreicht werden.

- SENSOCEL® Dentalpartikel sorgen für eine effektive, aber schonende Reinigung.

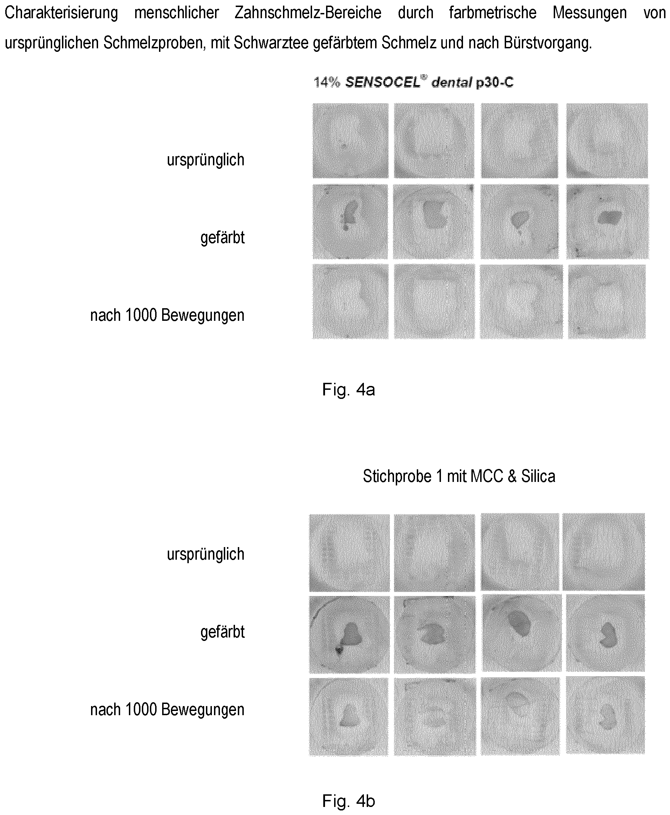

Farbmetrische Messungen werden in den Fig. 4a und 4b veranschaulicht.

<u>B) Zum Abriebwert:</u>

**Relativer Dentin-Abrieb (RDA)**

**Versuchsaufbau:**

- Herstellung einer Zahnpastaformel mit insgesamt 14 % verschiedener Reinigungspartikel (auf Wasserbasis)

- Bestimmung von RDA (relativer Dentin-Abrieb) anhand erhaltenem dritten molaren humanen Dentin (in vitro) basierend auf DIN EN ISO 11609: 2010 (Anlage B)

- Dentinschnitte wurden mit verminderter Körnung (4000er Körnung - 6 um) für eine beliebige Oberfläche geschliffen und mit einem Probenhalter fixiert.

- Suspension / Slurry von 25 g Zahnpasta in 40 mL Wasser, Erneuerung des Slurry nach 30 min.

- Einsatz des Bürstensinnulators ZM-3_8 (SD-Mechatronic, Deutschland), Zahnbürste Oral-B Indicator 35 medium (Druck 150 g, 10.000 Hübe bei 60 mm Nabe 1 cm), 6 Messungen je Probe

- profilometrische Bestimmung von Dentin-Verlust durch Profilometrie

$$RDA = \frac{\text{mittlerer Abrieb der getesteten Zahnpasta}}{\text{mittlerer Abrieb der getesteten Referenz}} * 100$$

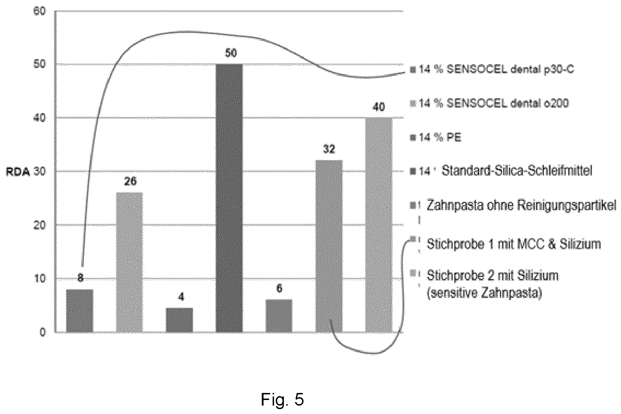

(Es wird auf die Fig. 5 verwiesen)

**Fazit**

- Alle Testformeln sowie zwei Stichproben bekannter Zahnpasta-Marken wurden nach dem oben beschriebenen Versuchsaufbau getestet.
- SENSOCEL ® Dentalpartien zeigen einen sehr geringen Abrieb im Vergleich zu Standard Silica-Schleifmitteln und verfügbaren Zahnpastamarken.
- SENSOCEL ® dental o200 weist einen etwas höheren RDA-Wert auf, der im Vergleich zu handelsüblichen Zahnpasten immer noch als niedriger RDA-Wert gilt.
- die gemessenen RDA-Werte für Stichprobe 1 und Stichprobe 2 korrelieren mit den Angaben des Herstellers (Stichprobe 1: RDA 36, Stichprobe 2: RDA 40).

B) Zum Abriebwert:

**Relative Dentin Abrasion (RDA)**

**Experimental set-up:**

- preparation of a toothpaste formula with in total 14 % of different cleansing particles (water-based)
- determination of RDA (relative dentin abrasion) on retained third molar human dentin (in vitro) based on **DIN EN ISO 11609: 2010 (Annex B)**
- dentin sections were ground at decreased grit (4000 Grit - 6 $\mu$m) for an even surface and were fixed on sample holder
- slurry preparation of 25 g toothpaste in 40 mL water, renewal of slurry after 30 min
- use of brushing simulator ZM-3.8 (SD-Mechatronic, Germany), toothbrush Oral-B Indicator 35 medium (pressure 150 g, 10,000 strokes at 60 mm sec$^{-1}$, hub 1 cm), 6 measurements per sample
- determination of dentin loss by profilometry

$$RDA = \frac{mean\ abrasion\ of\ the\ tested\ toothpaste}{mean\ abrasion\ of\ the\ tested\ reference} * 100$$

**Conclusion**

- all test formulas es well as two market samples of well-known toothpaste brands have been tested according to the above described experimental set-up
- *SENSOCEL*® **dental** particles show a very low abrasion compared to Standard Silica abrasive and available toothpaste brands
- *SENSOCEL*® *dental* o200 shows a slightly higher RDA-value which is still considered as low RDA-value compared to commercially available toothpastes
- the RDA values measured for market sample 1 and market sample 2 correlate with the manufacturer specifications (market sample 1: RDA 36, market sample 2: RDA 40)

**Patentansprüche**

1. Mund- und Zahnpflege- sowie Zahnreinigungsmittel in Form einer Paste mit einem Zusatz von feinkörnigen, wasserunlöslichen Reinigungskörpern, **dadurch gekennzeichnet, dass** die Reinigungskörper aus reiner nativer Cellulose bestehen, die einer unbehandelten, natürlichen Cellulose entspricht, die mittels Aufschlussverfahren aus pflanzlichem Vorläufermaterial gewonnenen wird und die im Gegensatz zu mikrokristalliner Cellulose keinem weiterem nachfolgendem chemisch-modifizierenden Prozess unterzogen wird, und als kurzfaserige Partikel mit einer unregelmäßigen Oberfläche und einer oberflächen-rauhen Struktur, die eine Vielzahl von Erhebungen und Vertiefungen aufweist, ausgebildet sind, und dass die Reinigungskörper eine durchschnittliche Partikelgröße von 30 $\mu$m bis 80 $\mu$m aufweisen.

2. Mund- und Zahnpflege- sowie Zahnreinigungsmittel Mittel nach Anspruch 1, **dadurch gekennzeichnet, dass** die Partikel der Reinigungskörper sphärische Partikel sind, die bezüglich ihres Durchmessers eine Partikelgröße von etwa 50 $\mu$m, aufweisen.

3. Mund- und Zahnpflege- sowie Zahnreinigungsmittel Mittel nach Anspruch 1 oder 2, **dadurch gekennzeichnet, dass** die Reinigungskörper aus reinen, weißen und geruchslosen Cellulose-Partikeln bestehen.

4. Mund- und Zahnpflege- sowie Zahnreinigungsmittel nach Anspruch 1 oder 2, **dadurch gekennzeichnet, dass** der Anteil der Reinigungskörper in der Paste 10 bis 20 Gew.-%, vorzugsweise 14 Gew.-%, beträgt.

5. Mund- und Zahnpflege- sowie Zahnreinigungsmittel nach einem der Ansprüche 1 bis 4 zur Verwendung in einer Methode für die Reinigung von Zähnen, einschließlich von mit Multibandapparaturen versehenen Zähnen.

6. Körper aus reiner nativer Cellulose zur Verwendung als Reinigungskörper zur Reinigung von Zähnen, insbesondere zur Reinigung von mit Multibandapparaturen versehenen Zähnen.

**Claims**

1. Oral and dental care and tooth cleaning means in the form of a paste with an addition of fine-grained, water-insoluble cleaning bodies, **characterized in that** the cleaning bodies consist of pure native cellulose which corresponds to an untreated, natural cellulose which is obtained from vegetable precursor material by means of a pulping process and which, in contrast to microcrystalline cellulose, is not subjected to any further subsequent chemical-modifying process, and which are in the form of short-fibered particles with an irregular surface and a surface-rough structure which has a large number of elevations and depressions, and **in that** the cleaning bodies have an average particle size of 30 $\mu$m to 80 $\mu$m.

2. Oral and dental care and tooth cleaning means according to claim 1, **characterized in that** the particles of the cleaning bodies are spherical particles which have a particle size of about 50 $\mu$m with respect to their diameter.

3. Oral and dental care and tooth cleaning means according to claim 1 or 2, **characterized in that** the cleaning bodies consist of pure, white and odourless cellulose particles.

4. Oral and dental care and dental cleaning means according to claim 1 or 2, **characterized in that** the proportion of cleaning agents in the paste is 10 to 20% by weight, preferably 14% by weight.

5. Oral and dental care and tooth cleaning means according to any one of claims 1 to 4 for use in cleaning teeth, including teeth provided with multiband appliances.

6. Body made of pure native cellulose for use as a cleaning body for cleaning teeth, in particular for cleaning teeth fitted with multi-band appliances.

**Revendications**

1. Produit d'hygiène bucco-dentaire et de nettoyage des dents sous forme de pâte avec une addition de corps nettoyants à grains fins, insolubles dans l'eau, **caractérisé en ce que** les corps nettoyants sont constitués de cellulose native pure, qui correspond à une cellulose naturelle non traitée, qui est obtenue par un procédé de digestion à partir d'un matériau précurseur végétal et qui, contrairement à la cellulose microcristalline, n'est soumise à aucun autre processus ultérieur de modification chimique, et sont réalisés sous forme de particules à fibres courtes avec une surface irrégulière et une structure rugueuse en surface qui présente une multitude de bosses et de creux, et **en ce que** les corps nettoyants présentent une taille moyenne de particules de 30 $\mu$m à 80 $\mu$m.

2. Produit d'hygiène bucco-dentaire et de nettoyage des dents selon la revendication 1, **caractérisé en ce que** d les particules des corps de nettoyage sont des particules sphériques, qui, en ce qui concerne leur diamètre, présentent une taille de particules d'environ 50 $\mu$m.

3. Produit d'hygiène bucco-dentaire et de nettoyage des dents selon la revendication 1 ou 2, **caractérisé en ce que** les corps nettoyants sont constitués de particules de cellulose pures, blanches et inodores.

4. Produit d'hygiène bucco-dentaire et de nettoyage des dents selon la revendication 1 ou 2, **caractérisé en ce que** la proportion des corps nettoyants dans la pâte est de 10 à 20 % en poids, de préférence de 14 % en poids.

5. Produit d'hygiène bucco-dentaire et de nettoyage des dents selon l'une quelconque des revendications 1 à 4, destinée à être utilisée pour le nettoyage des dents, y compris des dents munies d'appareils multibagues.

6. Corps en cellulose native pure à utiliser comme corps nettoyant pour le nettoyage des dents, en particulier pour le nettoyage des dents munies d'appareils multibagues.

Fig. 1

Fig. 2

Fig. 3

Charakterisierung menschlicher Zahnschmelz-Bereiche durch farbmetrische Messungen von ursprünglichen Schmelzproben, mit Schwarztee gefärbtem Schmelz und nach Bürstvorgang.

14% SENSOCEL® dental p30-C

ursprünglich

gefärbt

nach 1000 Bewegungen

Fig. 4a

Stichprobe 1 mit MCC & Silica

ursprünglich

gefärbt

nach 1000 Bewegungen

Fig. 4b

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102014201628 A1

**In der Beschreibung aufgeführte Nicht-Patentliteratur**

- **STOOKEY. G.** ; **MUHLER, J.** Laboratory Studies Concerning the Enamel and Dentin Abrasion Properties of Common Dentifrice Polishing Agents.. *J. Dent. Res.*, 1968, vol. 47 (4), 524-532
- **STOOKEY, G.** ; **BURKHARD, T.** ; **SCHEMEHORN, B**. In Vitro Removal of Stain with Dentifries.. *J. Dent. Res.*, 1982, vol. 61 (11), 1236-1239
- **ADDY, M.** ; **MORAN, J.** Mechanisms of stain formation on teeth, in particular associated with metal ions and antiseptics. *Adv. Dent. Res*, 1995, vol. 9 (4), 450-456